# FASCICULE DE BREVET EUROPEEN

(11) **EP 0 934 342 B1**
(45) Date de publication et mention de la délivrance du brevet: **02.01.2002**
(21) Numéro de dépôt: 97912239.7
(22) Date de dépôt: 23.10.1997
(51) Int. Cl.: C08B 37/08, C12N 15/00

(54) **COMPOSITION CONTENANT DU CHITOSAN**
CHITOSAN ENTHALTENDE ZUSAMMENSETZUNG
COMPOSITION CONTAINING CHITOSAN

(30) Priorité: 23.10.1996 FR 9612894; 26.02.1997 FR 9702296
(43) Date de publication de la demande: 11.08.1999
(73) Titulaire: TRANSGENE S.A., 67000 Strasbourg (FR)
(72) Inventeur: KOLBE, Hanno, F-67204 Achenheim (FR)
(74) Mandataire: Warcoin, Jacques
(86) Numéro de dépôt international: FR9701897
(87) Numéro de publication internationale: WO9817693

(56) Documents cités:
- US-A- 4 970 150
- CHEMICAL ABSTRACTS, vol. 115, no. 21, 25 novembre 1991 Columbus, Ohio, US; abstract no. 225479, "Molecular weight-low chitosan as gene carrier in molecular cloning" XP002034422 & JP 03 198 782 A (VITAMIN KENKYUSHO K.K.) 29 août 1991
- PATENT ABSTRACTS OF JAPAN vol. 010, no. 172 (C-354), 18 juin 1986 & JP 61 021103 A (YAIZU SUISAN KAGAKU KOGYO KK), 29 janvier 1986,
- A. DOMARD ET AL.: "Glucosamine oligomers: 1. Preparation and characterization" INTERNATIONAL JOURNAL OF BIOLOGICAL MACROMOLECULES, vol. 11, no. 5, 1989, GB, pages 297-302, XP002034421
- PATENT ABSTRACTS OF JAPAN vol. 012, no. 186 (C-500), 31 mai 1988 & JP 62 292802 A (HIGETA SHOYU KK), 19 décembre 1987,

## Description

La présente invention a pour objet des composés de chitosan comprenant de 5 à 300 répétitions d'unités glucosamines et une composition pharmaceutique comprenant de tels composés. Plus particulièrement, la présente invention concerne un procédé de préparation de chitosan selon l'invention et son utilisation pour le transfert d'un acide nucléique dans une cellule hôte.

Le transfert de gènes dans une cellule donnée est à la base même de la thérapie génique. Cette nouvelle technologie dont le champ d'application est vaste, permet d'envisager le traitement de maladies graves pour lesquelles les alternatives thérapeutiques classiques sont peu efficaces voire inexistantes et concerne aussi bien les maladies génétiques (hémophilie, mucoviscidose, myopathie ...) qu'acquises (cancer, syndrome d'immunodéficience acquise SIDA...).

L'approche la plus pratiquée consiste à utiliser un vecteur viral pour introduire l'acide nucléique thérapeutique dans les cellules à traiter et, en particulier rétroviral et adénoviral. En effet, les virus ont développé des mécanismes sophistiqués pour traverser les membranes cellulaires, échapper à la dégradation au niveau des lysosomes et faire pénétrer leur génome dans les noyaux afin d'assurer l'expression du gène thérapeutique. Cependant, l'approche virale a ses limitations, notamment une capacité de clonage restreinte, une production potentielle de particules virales compétentes pour la replication susceptibles de dissemination dans l'organisme hôte et l'environnement, un risque de mutagénèse insertionnelle dans le cas des vecteurs rétroviraux et une induction de réponses immunitaires et inflammatoires chez l'hôte qui entravent les répétitions de traitement dans le cas des vecteurs adénoviraux. Ces inconvénients importants pour un usage humain justifient la recherche de systèmes alternatifs de transfert d'acides nucléiques. A cet égard, de nombreux composés amphiphiles et cationiques ont déjà été décrits dans la litérature (voir par exemple Behr et al. 1994, Bioconj Chem 5, 382-389).

Dans cette optique, il peut également être avantageux d'avoir recours à des polymères (répétition d'unités monomèriques). Cependant, les polymères protéiques sont à éviter du fait de leur nature potentiellement immunogène. Les polymères neutres tels que les dextrans, polyvinylpyrrolidone (PVP) et polyéthylèneglycol (PEG) ont l'avantage d'être peu toxiques in vivo mais, du fait de l'absence de charges positives, ne complexent pas les acides nucléiques de façon satisfaisante. Enfin, les polymères cationiques, tels que les dendrimères (WO 95/24221), le polyéthylène imine (WO 96/02655) et la polylysine (WO 95/33061) pourraient présenter une activité hémolytique après administration intraveineuse.

Un autre polymère cationique est constitué par le chitosan, un polysaccharide naturel formé par la répétition linéaire de résidus de glucosamine. Il existe d'ores et déjà des préparations commerciales de chitosan obtenues par déacylation de la chitine. D'une manière générale, elles comportent une longue chaîne d'unités de glucosamine et présentent une masse moléculaire moyenne élevée pouvant atteindre 2000 kDa. A titre indicatif, les préparations de plus faible masse moléculaire disponibles à l'heure actuelle ont environ 70 kDa et comportent en moyenne 430 résidus glucosamine (une unité glucosamine à une masse moléculaire de 161 Da).

Quelques documents de l'art antérieur reportent l'usage du chitosan pour le transfert de substances médicamenteuses et de polypeptides (Berscht et al., 1994, Biomaterials 15, 593-600 ; Chandry et Sharma, 1990, Biomater. Artif Cells Artif Organs 18, 1-24 et Illum et al., 1994, Pharm. Res. 11, 1186-1189) et plus récemment d'acides nucléiques (Murata et al., 1996, Carbohydrate Polymers 29, 69-74). Ce dernier document décrit la mise en oeuvre de chitosan dont les résidus amines sont quaternisés par triméthylation et conjugué à du galactose pour cibler plus particulièrement les cellules hépatiques. Ce document ne mentionne pas la possibilité de lier l'ADN sans quaternisation préalable.

Le document JP-A-03 198 782 divulgue l'introduction de gènes dans des cellules en utilisant du chitosan de faible poids moléculaire comme agent de transfert. Le chitosan de faible poids moléculaire a de préférence une viscosité intrinsèque de 0,1-1,0 dl/g. Dans ce document, les résidus chitosan sont différents de ceux mis en oeuvre dans la présente invention.

En outre, les préparations de l'état de la technique présentent certains inconvénients qui compromettent leur utilisation pour le transfert de gènes, notamment chez l'homme. En particulier, elles contiennent des pigments et d'autres contaminants qui même en faible quantité peuvent être nocifs. De plus, leur solubilité dans l'eau ou les tampons de conditionnement acceptables d'un point de vue pharmaceutique est médiocre et nécessite l'ajout d'au moins 0,1 % d'acide acétique et la solution obtenue reste visqueuse. Enfin, il est fort probable que la complexation de ces longues chaînes de polycations (en moyenne 430 unités pour les préparations de plus faible masse moléculaire) avec une longue chaîne de polyanions (acide nucléique) favorise les liaisons interchaînes et donc la formation de complexes de grande taille susceptibles de précipiter en solution. La présente invention apporte une solution avantageuse à ces différents problèmes.

On a maintenant généré par hydrolyse acide, des fragments de chitosan de faible masse moléculaire : inférieure à 5 kDa, comprise entre 5 et 10 kDa et supérieure à 10 kDa, et montré leurs propriétés particulièrement avantageuses pour le transfert de gènes. Les fragments de chitosan de la présente invention ont une taille suffisante pour former avec l'acide nucléique thérapeutique un complexe d'une stabilité adéquate pour permettre son transport dans la cellule hôte mais également sa libération in vivo. En outre, le procédé mis en oeuvre permet de réduire les pigments qui contaminent les préparations de l'art antérieur et d'améliorer la solubilité dans l'eau.

C'est pourquoi la présente invention a pour objet une composition comprenant un composé de formule générale [X]n dans laquelle n représente un nombre entier de 5 à 300 et X a la formule (I) suivante : 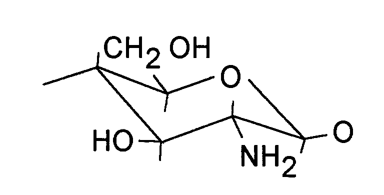 et une substance thérapeutiquement active, caractérisé en ce que tout ou partie des résidus X composant ledit composé est modifié par N-, C- et/ou O-alkylation, alcylation, -amino-alkylation et/ou -polyoxyéthylénation.
Un premier objet de l'invention concerne une composition comprenant un composé comprenant une répétition linéaire de 5 à 300 résidus glucosamines de formule (I) telle que définie ci-dessus. Ce composé selon l'invention est avantageusement utilisé pour son pouvoir transfectant pour le transfert d'un gène ou d'un vecteur d'intérêt dans une cellule hôte. On préfère tout particulièrement le cas où deux résidus de glucosamine sont liés de la manière suivante :

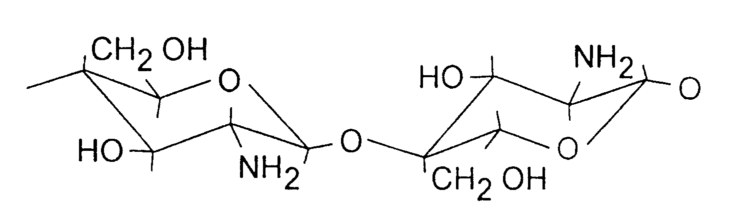

De manière avantageuse, dans la formule générale [X]n, n représente un nombre entier de 5 à 150, de manière préférée, de 5 à 75 et, de manière tout à fait préférée, n est compris entre 5 et 50.

Les composés particulièrement préférés dans le cadre de la présente invention sont les suivants :
(i) un composé présentant une masse moléculaire moyenne inférieure à 5 kDa,
(ii) un composé présentant une masse moléculaire moyenne comprise entre 5 et 10 kDa, et
(iii) un composé présentant une masse moléculaire moyenne supérieure à 10 kDa et inférieure à 50 kDa.

Bien entendu, un composé utilisable selon l'invention peut être modifié chimiquement par addition, délétion et/ou substitution de radicaux ou groupements chimiques de manière à améliorer sa stabilité, sa demi-vie, la complexation avec l'acide nucléique et/ou son pouvoir transfectant. La modification peut porter, sur une partie (modification partielle) ou l'ensemble (modification complète) des résidus glucosamines et sur un ou plusieurs radical(aux) de chacun des résidus glucosamines. Selon un mode de réalisation avantageux, un composé utilisable selon l'invention est modifié par N-, C- et/ou O-alkylation, -acylation, -amino-alkylation et/ou - polyoxyéthylénation (voir par exemple Dunn et al., 1993, J. Applied Polymer Science 50, 353-365 ; Boullanger et al., 1995, Carbohydr. Res. 278, 91-101 ; Muzzarelli et al., 1983, J. Memb. Science 16, 295-308). A cet égard, les composés particulièrement préférés sont la poly-N-dodecyl-polyglucosamine dans lequel le radical NH2 de la formule (I) est remplacé par NH-(CH2)11-CH3 et la mono-Cα-octadecylpolyglucosamine dans lequel le radical OH du composé selon l'invention en bout de chaîne est remplacé par O-(CH2)17-CH3. Mais il est également possible de greffer un résidu alkyl de type hexyl à octadécyl ou un résidu alkyl mono non saturé de type oléoyl.

De manière particulièrement avantageuse, le composé utilisable selon l'invention est un sel de chitosanium, notamment un sulphate, un phosphate et, plus préférentiellement, un chlorure.

Les composés utilisables selon l'invention peuvent être obtenus de différentes manières. Ils peuvent par exemple être synthétisés chimiquement à partir du monomère de glucosamine par polymérisation ou à partir d'une préparation de haute masse moléculaire par hydrolyse enzymatique ménagée. Mais on préfère tout particulièrement mettre en oeuvre un procédé d'hydrolyse acide des préparations de chitosan de l'état de la technique suivi d'une diafiltration différentielle, tel que décrit ci-après.

La présente invention concerne également le composé de formule [X]n dans laquelle n représente un nombre entier de 5 à 300 et X a la formule (1) suivante : 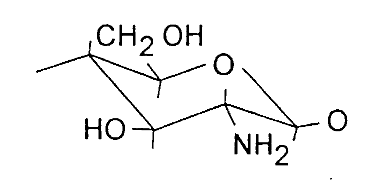 caractérisé en ce qu'il est présent sous forme de sel et plus particulièrement, de chlorure, de sulfate ou de phosphate ; ces composés préférés correspondant aux utilisations décrites précédemment.

La présente invention concerne également une préparation substantiellement pure de chitosan. Elle est en particulier obtenue par toute technique de purification classique et notamment, par extraction des pigments et autres contaminants résiduels. Cette étape de purification peut être réalisée sur les préparations commerciales de chitosan pour générer une préparation substantiellement pure de masse moléculaire élevée ou non qui peut être utilisée pour complexer un acide nucléique.

La présente invention concerne plus particulièrement une composition comprenant au moins un composé ou une préparation selon l'invention et une substance thérapeutiquement active. Il s'agit préférentiellement d'une substance chargée négativement et, en particulier, d'un acide nucléique. Par acide nucléique, on entend aussi bien un acide désoxyribonucléique (ADN) qu'un acide ribonucléique (ARN) sens ou antisens. Par ailleurs, l'acide nucléique peut être homologue ou hétérologue à la cellule hôte. Il peut s'agir de séquences naturelles, hybrides ou synthétiques dérivées d'ADN génomique, d'ADNc, d'ARNm, d'ARNr, d'ARNt, d'ARN viral, d'une origine quelconque (procaryote, eucaryote, virus, parasite, plante ...). Elles peuvent être obtenues par les techniques conventionnelles de biologie moléculaire (clonage, PCR pour Polymerase Chain Reaction en anglais ...) ou par synthèse chimique.

Selon un mode de réalisation particulier, l'acide nucléique mis en oeuvre dans le cadre de la présente invention est un vecteur pour l'expression d'un gène d'intérêt thérapeutique. Le vecteur d'expression est avantageusement sous forme de plasmide mais on peut également avoir recours à un vecteur viral (dérivé d'un rétrovirus, adénovirus, poxvirus, virus de l'herpès, virus associé à l'adénovirus ...). S'agissant du mode préféré, le choix des plasmides utilisables dans la présente invention est vaste. On peut employer un plasmide/vecteur de l'état de la technique ou le construire par les techniques de manipulations génétiques (Maniatis et al., 1989, Laboratory Manual, Cold Spring Harbor Laboratory Press, Cold Spring Harbor NY). Avantageusement, il possède les éléments génétiques lui permettant de se repliquer et de se maintenir dans un microorganisme et/ou une cellule hôte, comme par exemple au moins une origine de replication et un gène de sélection. Dans ce dernier cas, il peut s'agir d'un gène codant pour une auxotrophie ou conférant une résistance à un antibiotique (ampicilline, tétracycline, puromycine, G418, chloramphénicol ...). Bien entendu, le plasmide en usage dans la présente invention peut également comprendre des éléments supplémentaires améliorant son maintien, sa stabilité dans la cellule hôte ou encore son intégration dans les chromosomes de l'hôte. D'une manière générale, de tels éléments sont connus de l'homme de l'art.

Conformément aux buts poursuivis par la présente invention, le gène d'intérêt thérapeutique en usage dans la présente invention peut coder pour un ribozyme, un ARN antisens ou encore un ARNm qui sera ensuite traduit en polypeptide. Il peut s'agir d'un polypeptide mature, d'un précurseur et notamment un précurseur destiné à être secrété et comprenant un peptide signal, d'un polypeptide natif, tronqué, chimère provenant de la fusion de séquences d'origines diverses ou d'un polypeptide muté présentant des propriétés biologiques améliorées et/ou modifiées. Parmi les polypeptides auxquels on peut envisager d'avoir recours, on peut citer plus particulièrement :
■ les cytokines ou lymphokines (interférons α, β et γ, interleukines et notamment l'IL-2, l'IL-6, l'IL-10 ou l'IL-12, facteurs nécrosant des tumeurs (TNF), facteurs stimulateurs de colonies (GM-CSF, C-CSF, M-CSF ...) ;
■ les récepteurs cellulaires ou nucléaires (récepteurs reconnus par des organismes pathogènes (virus, bactéries, ou parasites) et, de préférence, par le virus VIH (Virus de l'Immunodéficience Humain) ou leurs ligands ;
■ les protéines capables de complémenter une activité cellulaire déficiente notamment dans le cadre d'une maladie génétique (facteur VII, facteur VIII, facteur IX, dystrophine ou minidystrophine, insuline, protéine CFTR (Cystic Fibrosis Transmembrane Conductance Regulator), hormones de croissance (hGH) ;
■ les enzymes (uréase, rénine, thrombine ...) ;
■ les inhibiteurs d'enzymes (α1-antitrypsine, antithrombine III, inhibiteurs de protéases virales ...) ;
■ les polypeptides à effet anti-tumoral capables d'inhiber au moins partiellement l'initiation ou la progression de tumeurs ou cancers (ARN anti-sens, anticorps, inhibiteurs agissant au niveau de la division cellulaire ou des signaux de transduction, produits d'expression des gènes suppresseurs de tumeurs, par exemple p53 ou Rb, polypeptides ou peptides stimulant le système immunitaire ...);
■ les protéines du complexe majeur d'histocompatibilité des classes I ou II ou protéines régulatrices agissant sur l'expression des gènes correspondants ;
■ les polypeptides capables d'inhiber une infection virale, bactérienne ou parasitaire et/ou son développement (polypeptides antigéniques ayant des propriétés immunogènes, épitopes antigéniques, anticorps, variants trans-dominants susceptibles d'inhiber l'action d'une protéine native par compétition ...) ;
■ les toxines (thymidine kinase de virus simplex de l'herpès 1 (TK-HSV-1), ricine, toxine cholérique, diphtérique ...) ou immunotoxines ; et
■ les marqueurs (β-galactosidase, luciférase ...).

Il est à signaler que cette liste n'est pas limitative et que d'autres gènes peuvent également être employés.

Avantageusement, le gène thérapeutique est placé sous le contrôle des éléments nécessaires à son expression dans la cellule hôte. Par "éléments nécessaires" on entend l'ensemble des éléments permettant sa transcription en ARN et la traduction d'un ARNm en polypeptide. Parmi ceux-ci, le promoteur revêt une importance particulière.

Il peut dériver d'un gène quelconque (eucaryote, viral, promoteur naturel du gène d'intérêt en question ...) ou peut être hybride. Par ailleurs, il peut être constitutif ou régulable. Alternativement, il peut être modifié de manière à améliorer l'activité promotrice, supprimer une région inhibitrice de la transcription, modifier son mode de régulation, introduire un site de restriction... On peut mentionner, à titre d'exemples, les promoteurs viraux CMV (Cytomegalovirus), du gène TK du virus HSV-1, précoce du virus SV40 (Simian Virus 40), adénoviral précoce (E1A) ou tardif (MLP pour Major Late Promoter) ou encore les promoteurs eucaryotes des gènes PGK (Phospho Glycerate kinase) murin ou humain, □1-antitrypsine (foie-spécifique), immunoglobulines (lymphocyte-spécifique).

Bien entendu, l'acide nucléique peut en outre comprendre des éléments additionnels tels que séquence intronique, séquence signal, séquence de localisation nucléaire, séquence terminatrice de la transcription, site d'initiation de la traduction de type IRES ou autre... On indique également que le vecteur peut comporter plusieurs gènes d'intérêt placés sous le contrôle d'éléments de régulation indépendants ou communs.

Selon un mode de réalisation optimal, les proportions respectives du composé selon l'invention et de l'acide nucléique sont de préférence déterminées de sorte que le rapport en charge dudit acide nucléique audit composé (R ADN/composé) est compris entre 1/10 et 10/1 et, de manière préférée entre 1/1 et 1/5.

Une composition selon l'invention peut être utilisée telle qu'elle ou en association avec d'autres composés et, en particulier un adjuvant capable d'améliorer son pouvoir transfectant. Les adjuvants utilisés préférentiellement dans la composition selon l'invention sont les composés amphiphiles cationiques et/ou les lipides neutres ou zwitterioniques. Dans le premier cas, on préfère un composé mono, di ou tri alkylé ou acylé portant de 1 à 4 charges positives tel qu'un lipide cationique. A titre d'exemples, on peut citer le 5-carboxyspermylglycine dioctadécylamide (DOGS), le 3β [N-(N', N'-diméthylaminoéthane)-carbamoyl] cholestérol (DC-Chol), le (2,3-droléylocyl-N-[2(sperminecarboxamido) éthyl] N, N-diméthyl-1-propanaminium trifluoroacétate) (DOSPA), la spermine cholestérol et la spermidine cholestérol. S'agissant d'un lipide neutre ou zwitterionique, on peut mentionner les phosphatidyl éthanolamine, phosphatidylcholine, phosphocholine, sphyngomyéline, céramide et cérébroside ou leurs dérivés. De préférence, il s'agit de la dioléyl phosphatidyl éthanolamine (DOPE).

Une composition selon l'invention est plus particulièrement caractérisée en ce que le rapport de charge lipide sur ADN est compris entre 25/1 et 1/1 et de préférence 5/1 et 2/1.

Il est également possible de combiner à la composition selon l'invention d'autres substances pour améliorer encore l'efficacité transfectionnelle ou la stabilité des complexes.

La présente invention concerne également l'utilisation d'une composition selon l'invention, pour préparer un médicament à but curatif, préventif ou vaccinal destiné au transfert d'acide nucléique dans une cellule hôte in vivo, ex vivo ou in vitro. Une composition selon l'invention peut être utilisée dans divers types de cellules hôtes, par exemple une cellule de microorganisme, de levure, d'insecte, de plante mais il s'agit de préférence d'une cellule de mammifère et, en particulier d'une cellule humaine. Ladite cellule peut être une cellule primaire ou tumorale d'une origine hématopoïétique (cellule souche totipotente, leucocyte, lymphocyte, monocyte, macrophage ...), hépatique, rénale, du système nerveux central, fibroblaste, épithéliale et notamment une cellule épithéliale pulmonaire, ou musculaire (myocyte, myoblaste, cardiomyocyte, cellule satellite ...).

Une composition selon l'invention peut être administrée par voie systémique ou par aérosol. On peut envisager plus particulièrement la voie gastrique, sous-cutanée, intracardiaque, intramusculaire, intraveineuse, intrapéritonéale, intratumorale, intrapulmonaire, intranasale ou intratrachéale. L'administration peut avoir lieu en dose unique ou répétée une ou plusieurs fois après un certain délai d'intervalle. La voie d'administration et le dosage appropriés varient en fonction de différents paramètres, par exemple de l'individu ou de la maladie à traiter ou encore du ou des gènes(s) à transférer. La formulation peut également inclure un excipient acceptable d'un point de vue pharmaceutique.

Une composition selon l'invention est en particulier destinée au traitement préventif ou curatif de maladies génétiques (hémophilie, mucoviscidose, myopathie de Duchenne et Becker ...), cancers et maladies virales (SIDA, infections à papillome, virus de l'herpès ...).

Enfin, la présente invention concerne également un procédé pour préparer un composé utilisable selon l'invention à partir d'une préparation de chitosan de haute masse moléculaire par hydrolyse acide, précipitation à l'éthanol et extraction des pigments en présence d'un solvant organique.

Les préparations de chitosan de haute masse moléculaire sont accessibles commercialement (par exemple Fluka, références 22741, 22742 et 22743). Dans le cadre de la présente invention, on préfère mettre en oeuvre une préparation d'une masse moléculaire moyenne d'environ 70 kDa qui correspond à la plus faible masse moléculaire disponible sur le marché (Fluka référence 22741). L'hydrolyse acide est réalisée en présence d'acide chlorhydrique à une molarité d'environ 4 M à une température d'environ 100°C et pendant environ 4 heures. Bien entendu, l'homme du métier est capable d'adapter les conditions expérimentales d'hydrolyse selon la préparation de départ. Après filtration grossière pour éliminer les insolubles et refroidissement, l'hydrolysat est précipité par de l'éthanol à une concentration finale d'environ 50% à froid. Le matériel précipité est récolté par centrifugation ou tout moyen classique. Il est préférable de procéder à plusieurs lavages dans de l'éthanol 50% avant de le reprendre dans de l'eau ou un tampon approprié.

Le matériel dissous est soumis à une extraction en présence de solvant. Tout solvant polaire et non miscible à l'eau peut être utilisé et, notamment, des alcools C₄ à C₈ et des esters de ces derniers. Un solvant préféré dans le cadre de l'invention est le butanol-2. Cette étape qui présente l'avantage de réduire considérablement les pigments contenus dans la préparation de départ, comprend de préférence une première extraction par un tiers de volume de butanol-2 puis une seconde extraction réalisée sur la phase aqueuse par un tiers de volume d'un mélange butanol-2/héxane (50/50).

Selon un mode de réalisation particulièrement avantageux, le procédé selon l'invention comprend une étape supplémentaire visant à séparer les composés de l'invention selon leur taille. Bien que différentes méthodes de fractionnement puissent être envisagées (gel perméation, filtration sur membrane ...), on préfère avoir recours à une diafiltration différentielle utilisant des membranes de seuil de coupure croissant. Le composé de l'invention ayant une masse moléculaire moyenne inférieure à 5 kDa est obtenu dans le filtrat résultant d'une première diafiltration à travers une membrane ayant un seuil de coupure de 5 kDa. Le concentré est alors récupéré et soumis à une seconde diafiltration à travers une membrane ayant un seuil de coupure de 10 kDa afin d'obtenir dans le filtrat le composé ayant une masse moléculaire moyenne de 5 à 10 kDa et, dans le concentré, le composé de l'invention ayant une masse moléculaire moyenne supérieure à 10 kDa et inférieure au chitosan de départ. Bien entendu, l'homme du métier est capable d'adapter la technique en fonction de la masse moléculaire moyenne désirée. Il est également possible de procéder à une ou plusieurs diafiltration(s) supplémentaire(s) sur le dernier concentré généré. Par exemple, le passage de ce dernier à travers une membrane ayant un seuil de coupure de 15 kDa permettra de récolter des composés selon l'invention ayant une masse moléculaire moyenne de 10 à 15 kDa.

La présente invention est plus particulièrement décrite en référence aux Figures suivantes :

La Figure 1 illustre un graphe de cytotoxicité sur cellules L132 obtenus avec les polymères pcTG12 NI, pcTG12 N2, pcTG12 N3, le dextran et la polylysine.

La Figure 2 illustre un graphe de cytotoxicité sur cellules CCRF-CEM obtenus avec les polymères pcTG12 NI, pcTG12 N2, pcTG12 N3, le dextran et la polylysine.

La Figure 3 illustre l'activité hémolytique testée sur des globules rouges de rat des polymères pcTG12 NI, pcTG12 N2, pcTG12 N3, du dextran et de la polylysine après 1 heure de contact.

La Figure 4 illustre l'activité hémolytique testée sur des globules rouges de rat des polymères pcTG12 N1, pcTG12 N2, pcTG12 N3, du dextran et de la polylysine après 5 heures de contact.

La Figure 5 illustre l'expression de la luciférase dans les cellules A549 transfectées avec le plasmide pTG11033 complexé à du DC Chol/DOPE (contrôle) ou du DC Chol/DOPE/chitosan à une concentration de 10, 30 et 60 %.

La Figure 6 illustre l'expression de la luciférase dans des myoblastes primaires transfectés avec le plasmide pTG11033 complexé à du DC Chol/DOPE (contrôle) ou du DC Chol/DOPE/chitosan à une concentration de 10, 30 et 60 %.

### EXEMPLES

### EXEMPLE 1 : préparation des fragments de chitosan.

De manière générale, les préparations commerciales de chitosan de masse moléculaire variée peuvent être utilisées et les plus appropriées à la mise en oeuvre de la présente invention, sont celles de faible masse moléculaire (par exemple la préparation Fluka sous la référence 22741 dont la masse moléculaire moyenne est d'environ 70 kDa). L'hydrolyse acide est réalisée dans une bouteille Schott DURAN de contenance 1 litre munie d'un agitateur magnétique. Après pesée, 25 g de chitosan (lot 340315/1 295) sont mis en présence de 625 ml d'acide chlorhydrique (HCI) 4 M (HCl de qualité analytique tel que R.P Normatur, Prolabo 20 252.290, min 36%). Après avoir procédé à l'échange de la phase gazeuse pendant 5 min avec de l'azote (pour éviter toute oxydation) et à la fermeture de la bouteille, le mélange réactionnel est placé sous agitation magnétique régulière (500 rpm) dans un bain d'huile maintenu à 100°C. Après équilibration de la température (15 min dans les conditions expérimentales précitées), on laisse la réaction se poursuivre pendant 4 heures à 100°C. On observe que ce traitement s'accompagne d'un changement de coloration de la solution qui apparaît brun clair. Bien entendu, les conditions d'hydrolyse sont adaptées à la préparation de chitosan de départ. Si celle-ci présente une masse moléculaire élevée (Fluka, 750 ou 2000 kDa), l'hydrolyse sera plus stringente (augmentation de la concentration en HCl ou du temps de réaction).

Le mélange réactionnel est immédiatement filtré (filtre de pores d'environ 0,6 mm) afin d'éliminer les impuretés et aggrégats insolubles. Le filtrat est transvasé dans une bouteille Schott DURAN de 2 litres et refroidi pendant environ 1 heure dans de la glace avant d'être précipité par ajout de 625 ml d'éthanol absolu (de qualité analytique, par exemple sds, référence 013A0516) et conservé une nuit à 2-4°C. Le lendemain, le matériel précipité est récolté par centrifugation (10000 g, 2-4°C, 10 min) et soumis à deux lavages dans un mélange éthanol/eau (50/50). Les culots sont repris dans 20 à 60 ml d'eau déminéralisée sur cartouche Milli Q (eau Milli Q), congelés à -80°C en attendant d'être lyophilisés. On obtient habituellement 12 à 16 g de fragments de chitosan sous forme de sels d'hydrochlorure.

On procède ensuite à l'extraction des pigments de la préparation de chitosan x nHCl de la façon suivante. 14,9 g de la préparation lyophilisée précédente sont dissous dans 300 ml d'eau Milli Q puis traités par 100 ml de butanol-2 (qualité analytique). Le mélange est alors agité vigoureusement et laissé à température ambiante dans une ampoule à décanter jusqu'à séparation des phases. La phase supérieure de coloration brunâtre est éliminée et la phase aqueuse est soumise à une nouvelle extraction en présence de 100 ml d'un mélange butanol-2/héxane (50/50). Comme précédemment, on récupère la phase inférieure dont on évapore le milieu aqueux par rotation sous vide.

Le matériel séché est finalement mis en solution dans 50 à 100 ml d'eau Milli Q et soumis à deux étapes de diafiltration consécutives mettant en oeuvre des membranes de seuil de coupure croissant afin de purifier des fragments de chitosan de masse moléculaire correspondante. La solution est tout d'abord filtrée à travers une membrane de seuil de coupure de 5 kDa (par exemple YM-5, Amicon). Cette dernière opération est répétée trois fois en complétant avec de l'eau Milli Q jusqu'au volume initial et les filtrats ainsi obtenus sont regroupés et congelés en attente d'une lyophilisation. On obtient une préparation sèche de couleur blanche de chlorure de chitosan d'une masse moléculaire inférieure à 5 kDa (désignés pcTG12 N1). Le concentré est quant à lui soumis à une seconde diafiltration sur membrane de seuil de coupure de 10 kDa (par exemple YM-10, Amicon). Après 3 lavages avec le volume initial, les filtrats sont regroupés, congelés et lyophilisés pour donner une préparation sèche de couleur blanche de chlorure de chitosan d'une masse moléculaire comprise entre 5 et 10 kDa (désignés pcTG12 N2). On récupère le concentré qui est également congelé et lyophilisé à partir duquel on obtient préparation sèche légèrement jaunâtre de chlorure de chitosan d'une masse moléculaire supérieure à 10 kDa (désignés pcTG12 N3).

Les préparations ainsi générées peuvent être caractérisées par les méthodes analytiques conventionnelles, par exemple par dosage des groupes amines libres (Curotto et Aros, 1993, Analytical Biochemistry 211, 240-241). On a également determiné la longueur des polymères par spectrométrie de masse desorption laser et on observe une distribution en courbe de Gausse. Les polymères de la préparation pcTG12 NI (< 5 kDa) sont formés d'une répétition de 5 à 35 résidus glucosamines, le maximum se situant aux environs de 10. Les polymères de la préparation pcTG12 N2 (5-10 kDa) présentent une répétition de 10 à 50 monomères, le maximum étant compris entre 30 et 35. Pour ce qui est de la préparation pcTG12 N3, on observe un nombre de résidus glucosamines supérieur à 15.

### EXEMPLE 2 : Complexation avec l'ADN et résultats sur gel

On évalue la capacité des préparations polymériques précédentes à complexer le plasmide reporter pCH11ON. Il comporte le bloc promoteur/enhancer du virus SV40 dirigeant l'expression du gène LacZ codant pour la β-galactosidase d'Escherichia coli munie en N-terminal d'un signal de localisation nucléaire issu de SV40.

500 ng d'ADN plasmidique sont mis en présence de quantités croissantes de chacune des préparations pcTG12 N selon un rapport en masse ADN/chitosan variant de 1/0,5 à 1/4 (les rapports ADN/chitosan précis sont : 1/0,5, 1/1, 1/1,5, 1/2, 1/3 et 1/4) dans un volume total de 10µl éventuellement complété par du TE (Tris 10 mM, EDTA 1 mM) pH 7,5. Le témoin négatif est constitué par de l'ADN plasmidique repris simplement dans du TE. Les mélanges sont analysés sur gel d'agarose 0,9% (tampon de migration TAEx1 pH 7,8 et migration 2 heures à 50V) et revelés par le bromure d'éthidium. L'ADN plasmidique libre migre sous la forme de plusieurs bandes correspondant aux différents topoisomères (ADN circulaire, superenroulé) alors qu'une fois compléxé au chitosan, il ne pénètre plus dans le gel et est visualisé dans les poches de dépôt.

Les résultats montrent que l'ADN plasmidique est entièrement compléxé aux polymères pcTG12 NI et N2 à partir d'un rapport ADN/chitosan de 1/2 et 1/3 respectivement. La formation du complexe est plus rapide avec les polymères de masse moléculaire supérieure à 10 kDa pour lesquels l'absence d'ADN libre est observée dès l'équimolarité (R 1/1). Dans leur ensemble, ces résultats montrent la capacité des 3 préparations de l'invention à lier l'ADN plasmidique.

### EXEMPLE 3 : Essais cytotoxiques

### A. In vitro

La cytotoxicité des polymères pcTG12 N1, N2 et N3 a été évaluée in vitro à l'égard des lignées cellulaires humaines L132 (ATCC CCL5) issues de poumons embryonnaires humains et CCRF-CEM (ATCC CCL119) issues d'un lymphoblastome humain. Ces lignées ont été retenues sur la base de leur comportement différent en culture cellulaire, la première poussant de manière adhérente (L132) et la seconde en suspension (CCRF-CEM).

Les cellules CCRF-CEM sont mises en culture dans une plaque de microtitration à 96 puits ayant un fond en forme de "v" (Costar) à raison de 1x104 cellules par puits et cultivées à 37°C en atmosphère CO₂ 5% dans du milieu RPMI 1640 (Gibco BRL) contenant 10 % de sérum de veau foetal (FCS) (Gibco BRL). Après 24 heures, les cellules sont centrifugées à 1000g pendant 10 min et le culot cellulaire est repris dans le même milieu en présence des polymères à tester (dissolution d'une quantité adéquate de chacun des polymères dans du milieu RPMI 1640-10% FCS de manière à obtenir une concentration finale de 0,1 µg/ml à 1 mg/ml puis filtration stérilisante sur filtres 0,2 µm (Acrodisk)). On utilise du milieu contenant du dextran de masse moléculaire 72 kDa (Sigma) à des concentrations identiques à celles utilisées pour les molécules pcTG12 à titre de témoin négatif et de la polylysine de masse moléculaire 30-70 kDa (Sigma) à titre de témoin positif. Les cellules contrôles sont cultivées dans les mêmes conditions mais en l'absence de polymères.

Les cellules L132 sont mises en culture dans une plaque de microtitration à 96 puits (Costar) à raison de 1x10⁴ cellules par puits et cultivées à 37°C en atmosphère CO₂ 5% dans du milieu E199 (Gibco BRL, référence 22340-012) contenant 5% de FCS. Après 24 heures, le surnageant de culture est remplacé par du milieu frais E199-5 % FCS dans lequel ont été dissous des concentrations appropriées de chacun des polymères de l'invention ou des témoins comme indiqué ci-dessus.

La viabilité des cellules L132 et CCRF-CEM est déterminée après 67 heures d'exposition aux différents polymères par le test MTT selon la technique décrite dans Sgouras et Ducan (1990, J. of Materials Science: Materials in Medecine 1, 61-68). Après une période d'incubation de 5 heures, le surnageant cellulaire est éliminé (éventuellement après centrifugation à basse vitesse dans le cas des lignées en suspension) et remplacé par 100 µl de DMSO de grade optique (Sigma) et on détermine l'absorbance à 550 nm au niveau de chaque puits de culture (lecteur de plaque Titerteck). Une faible absorbance est signe d'une mortalité importante. La viabilité est exprimée en pourcentage par rapport à l'absorbance lue pour les cellules cultivées en absence de polymères.

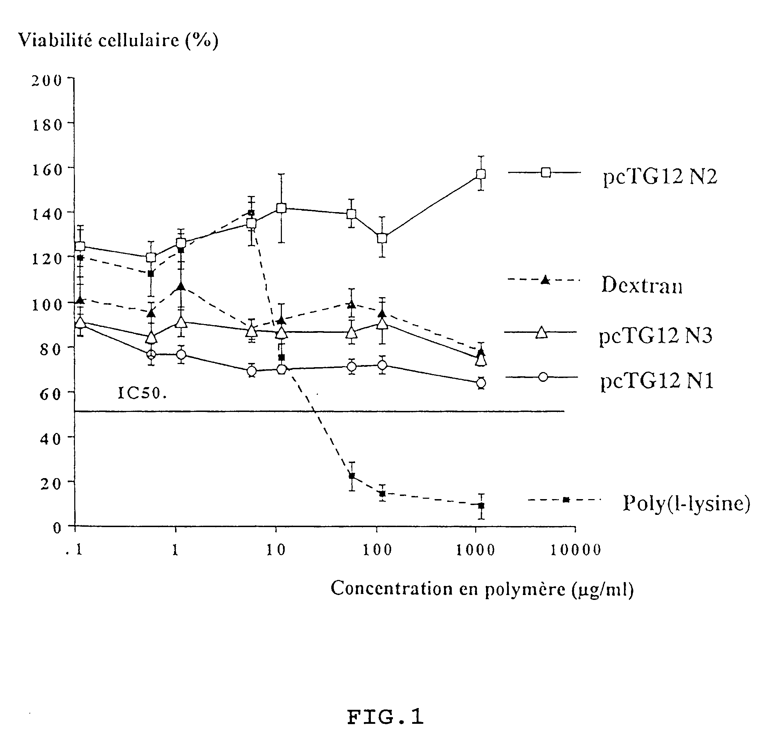

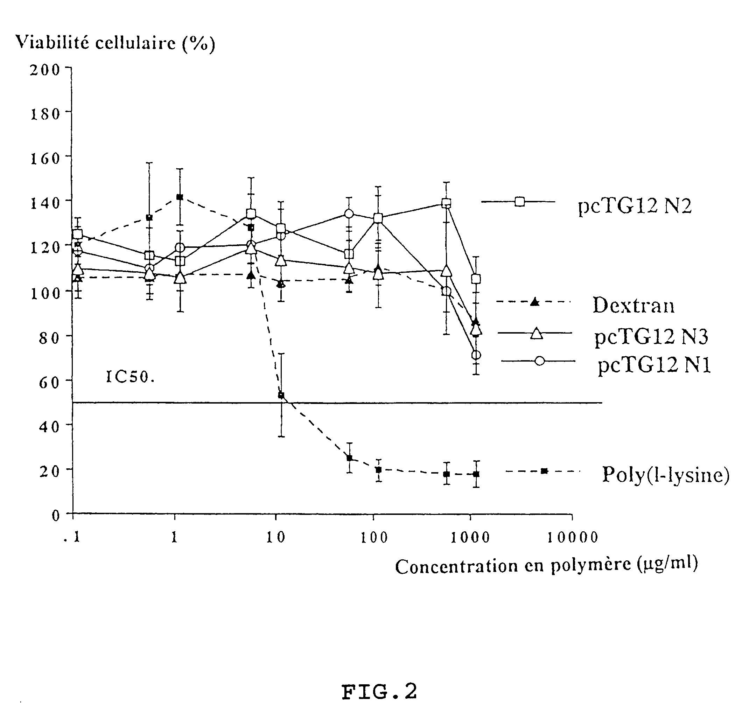

Les Figures 1 et 2 présentent les résultats obtenus pour les cellules L132 et CCRF-CEM respectivement et montrent que les 3 polymères selon l'invention (fragments de chitosan de masse moléculaire <5, 5-10 et > 10 kDa) affectent peu la viabilité cellulaire et ceci à des doses aussi élevées que 0,5 mg/ml, tout comme le dextran (non toxicité reconnue). A 1 mg/ml, on observe une réduction de la viabilité d'un facteur environ 20%. L'IC50 soit 50 % de cellules viables correspond à une concentration d'environ 2 mg/ml. En revanche, la polylysine excerce un effet cytotoxique à des doses 100 fois inférieures (IC50 atteinte à une concentration de 10 µg/ml).

Dans leur ensemble, ces données confirment l'absence de cytotoxicité des préparations de chitosan de l'invention prérequise à leur utilisation pour le transfert de matériel génétique chez l'homme.

Des données récemment publiées (Carrino-Gomez et Ducan, 1996,Proc. 1st International Symposium on Polymer Therapeutics : From the Laboratory to Clinical Practise. Janvier 10-12 1996, Londres) semblent indiquer une certaine cytotoxicité des préparations de chitosan de masse moléculaire élevée. Cet effet cytotoxique pourrait être dû à la masse moléculaire ou à un effet de charge. On indique également que le conjugué chitosan-galactose décrit par Murata et al. (1996, supra) présente une activité cytotoxique supérieure aux composés de la présente invention (50 % de cytotoxicité pour environ 200 µg/ml si l'on se reporte à la Figure 1).

### B. In vivo

200 µg de chacune des préparations de chitosan de l'invention ont été administrés à des lapins par voie intraveineuse et on n'observe aucun effet toxique.

### EXEMPLE 4 : Essais d'hématocompatibilité

### A. Hémolyse de globules rouges

Les polymères pcTG12 N1, N2 et N3 sont dilués à des concentrations allant de 1 à 10⁷ ng/ml dans du tampon PBS, mis en présence d'une suspension fraîche de globules rouges de rat (2 % poids/volume) et incubés à 37°C pendant 1 et 5 heures. Le mélange est centrifugé à 1500g pendant 10 min à température ambiante et la quantité d'hémoglobine présente dans le surnageant est évaluée par dosage spectophotométrique à 550 nm. On procède de même avec les témoins dextran et polylysine. Les résultats sont exprimés en pourcentage de lyse par rapport à un contrôle traité par du Triton X-100 (Sigma) qui correspond à 100 % de lyse.

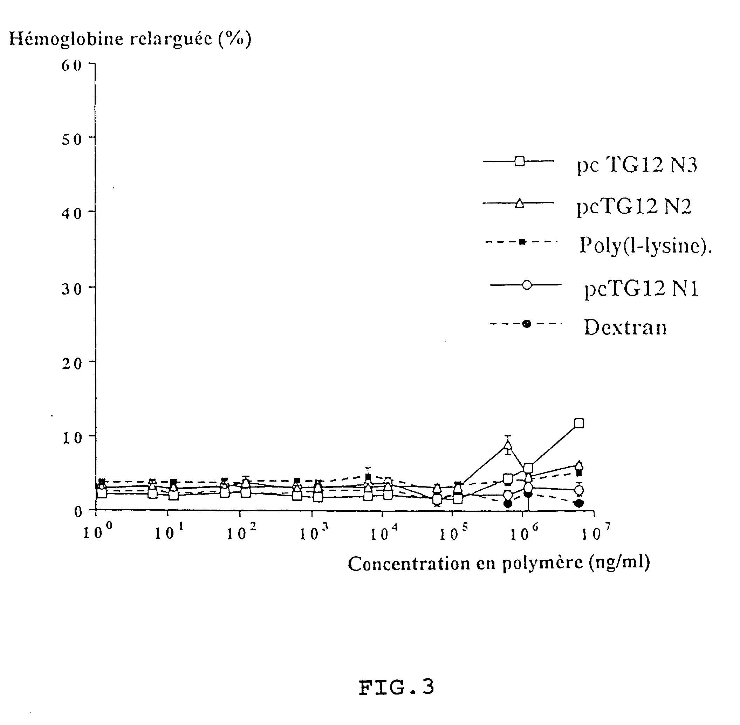

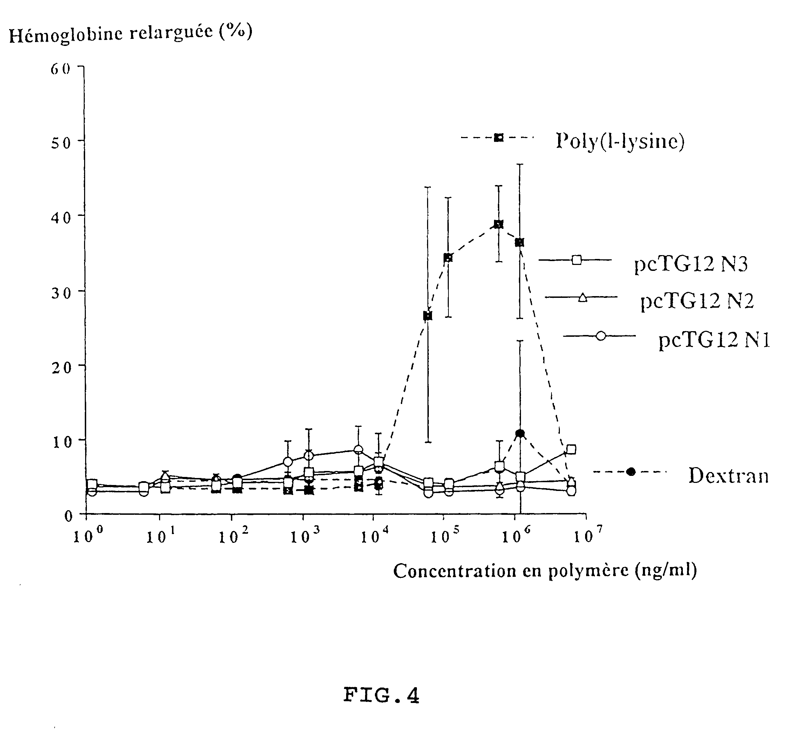

Les données obtenues après 1 et 5 heures de contact sont illustrées par les Figures 3 et 4. Les trois polymères de l'invention ne montrent pas d'activité membranaire jusqu'à 100 µg/ml (10₅ ng/ml) et au delà une activité lytique inférieure à 10 % après 5 heures de contact. Dans les mêmes conditions, la polylysine à une concentration supérieure à 10 µg/ml affecte l'intégrité membranaire.

### B. Eludes de microcopie électronique

Les hématies de rats sont incubées pendant 1 ou 5 heures en présence de 10 µg/ml de pcTG12 N1, N2, N3, dextran ou polylysine puis mises en suspension une nuit dans une solution de glutaraldéhyde (0,25 % vol/vol) reconstituée dans du PBS (Oxoide). Les cellules sont centrifugées à faible vitesse (1200g 1 min à température ambiante ; centrifugeuse MSE Microcentour) et, après élimination du surnageant, reprises dans du tétroxyde d'osmium 1 % (poids/vol) dans du PBS. Après 1 heure à température ambiante, on procède à une centrifugation dans les mêmes conditions que précédemment et les cellules sont traitées par de l'éthanol 50 % dilué dans du PBS pendant 30 min. Cette dernière opération est répétée avec de l'éthanol 60 %, 70 %, 80 %, 90 % et enfin de l'éthanol absolu. Le culot cellulaire est mis en suspension dans de l'héxamethyldisilazane (HMDS, Sigma) et, après évaporation du HMDS, les échantillons sont recouverts d'or (50 µA EMTECH Gold Coater). L'examen en microscopie électronique (Philips XL) montre que les polymères de l'invention ne perturbent pas la morphologie des hématies (morphologie comparable aux cellules traitées par du PBS seul ou du dextran) alors qu'un grand pourcentage des cellules incubées en présence de polylysine présente une morphologie altérée (en forme d'étoile).

### EXEMPLE 5 : Transfection des complexes chitosan/acide nucléique

Le plasmide pTG11033 (1 mg/ml) est utilisé pour évaluer l'efficacité transfectionnelle des composés de l'invention. Il comporte le gène reporter luc qui code pour la luciférase placé sous le contrôle du promoteur CMV et le gène de résistance à la kanamycine. Mais tout autre plasmide peut également être employé. Le DC-Chol est obtenu par synthèse chimique à une échelle de 20 g selon le procédé décrit par Gao et al. (1991, BBRC 179, 280-285). Le colipide DOPE est disponible commercialement (Sigma, référence P5078). Les solutions stock des préparations de chitosan pcTG12N sont conservées à 10 mg/ml dans du chloroforme. Le mélange DC-Chol/DOPE est obtenu par mélange des deux composés selon un rapport en poids de 1/1 et une solution stock est établie dans du chloroforme à raison de 10 mg/ml.

### A. Transfection de cellules pulmonaires

Les transfections sont réalisées en plaques de 96 puits dans lesquels sont mises en culture 2x104 cellules A 549 (ATCC CCL185) dérivées de carcinome pulmonaire humain , dans du milieu Eagle modifié par Dulbecco (DMEM) contenant 10 % de FCS. Après 24 heures de culture à 37°C en atmosphère C02 (5 %), les cellules sont mises en présence de complexes ADN/DC Chol/DOPE en absence ou en présence des polymères pcTG12 N1. La préparation des complexes se fait dans des tubes Nunc en polypropylène et sous hotte à flux laminaire afin de préserver la stérilité des préparations. Brièvement, on procède tout d'abord au mélange des lipides au polymère par injection du DC-Chol/DOPE soit dans de l'eau (témoin 0 % de chitosan) ou dans de l'eau additionnée de chitosan (10, 30 ou 60 %) à l'aide d'une seringue Hamilton et en maintenant l'ensemble sous agitation lente. On indique que les pourcentages de chitosan sont calculés par rapport à la charge apportée par le lipide cationique DC-Chol. L'ADN est ensuite ajouté au mélange lipide/polymère.

Plus précisemment 2 mg de DC-Chol/DOPE (200 µl de solution stock) sont séchés et les lipides secs repris dans 150 µl d'éthanol jusqu'à dissolution complète (une sonication de 10 secondes peut améliorer la dissolution) et on génère les mélanges suivants :
DC Chol/DOPE/ADN : 40,5 µl (540,5 µg) de la solution lipidique précédente sont ajoutés à 500 □l d'eau dont 455 µl (soit 455 µg) sont mis en présence de 30 µg de pTG11033 dissous dans un volume de 145 µl d'eau,
DC Chol/DOPE/10 % Chitosan/ADN : 40,5 µl (540,5 µg) de la solution lipidique précédente sont ajoutés à 498 µl d'eau et 2 µl (20 µg) de chitosan dont 455 µl sont mis en présence de 30 µg de pTG11033 dissous dans un volume de 145 µl d'eau,
DC Chol/DOPE/30 % Chitosan/ADN : 40,5 µl (540,5 µg) de la solution lipidique précédente sont ajoutés à 494 µl d'eau et 6 µl (60 µg) de chitosan dont 455 µl sont mis en présence de 30 µg de pTG11033 dissous dans un volume de 145 µl d'eau,
DC Chol/DOPE/60 % Chitosan/ADN : 40,5 µl (540,5 µg) de la solution lipidique précédente sont ajoutés à 488 µl d'eau et 12 µl (120 µg) de chitosan dont 455 µl sont mis en présence de 30 µg de pTG11033 dissous dans un volume de 145 µl d'eau.

Après 24 heures à 4°C, on ajoute aux mélanges précédents 19 µl de NaCl 5 M afin d'ajuster la concentration à 0,9 % (154 mM). On prélève 86 µl de chacun des mélanges ainsi générés qui sont placés dans la ligne A d'une plaque de microtitration de 96 puits, complétés à 100 µl à l'aide du milieu DMEM et dilués au 1/2 en cascade dans du milieu DMEM. Le volume dans chaque puit est complété à 235 µl avec le milieu de culture et 225 µl prélevés et ajoutés aux cellules A549 en culture depuis 24 heures. 25 µl de FCS sont ajoutés 4 heures après le début de la transfection. La culture est poursuivie pendant 48 heures, le milieu éliminé et après avoir été lavées par 100 µl de tampon PBS, les cellules sont lysées par 50 µl de tampon de lyse (Promega) et congelées à -80°C jusqu'à mesure de l'activité luciférase. L'activité luciférase est révélée sur 20 µl (soit 2/5 de l'échantillon) par mesure de l'émission de photons à l'aide d'un luminomètre (LB96P, Berthold). Les résultats sont exprimés en RLU/mn/échantillon. A titre indicatif, la concentration en protéine de l'échantillon mesuré est de 24 à 32 µg.

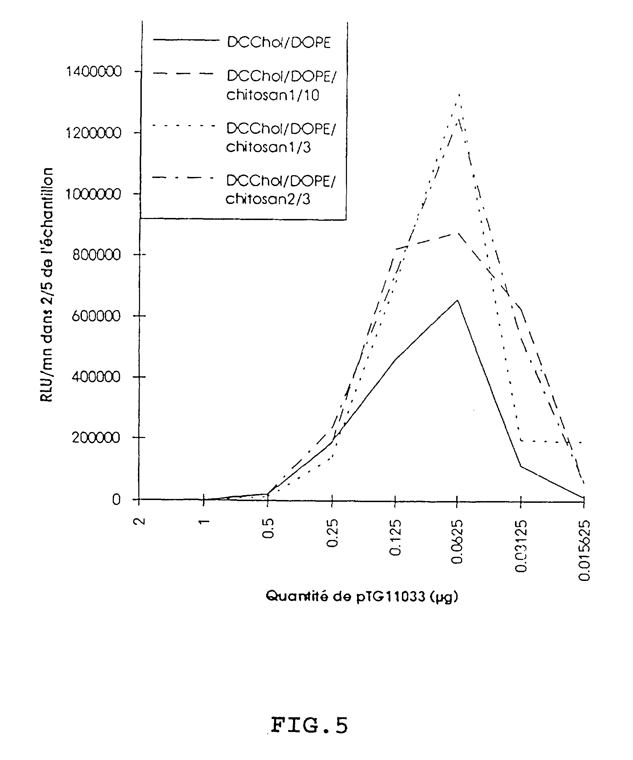

Comme montré dans la Figure 5, la présence de chitosan dans les complexes DC Chol/DOPE/ADN s'avère avantageuse, le taux de transfection reflété par l'activité luciférase étant supérieur avec les complexes contenant du chitosan qu'avec les complexes qui en sont dépourvus. En effet, l'activité luciférase est significativement plus élevée d'un facteur environ 1,5 en présence de 10% de chitosan et d'un facteur 2 en présence de quantités plus importantes (30 et 60%).

### B. Transfection de myoblastes primaires

Les myoblastes sont obtenus de biopsies pratiquées sur des muscles quadriceps de chiens mâles. Les prélèvements sont fragmentés en segments d'environ 1 mm³ et mis en culture dans des boîtes de Pétri (Falcon) recouvertes de gélatine plasmatique humaine 1 %. La culture est effectuée dans du milieu Ham F14 (Gibco BRL) additionné de facteurs de croissance [Glutamine 2 mM (BioMérieux), FGF 10 ng/ml (Peprotech-TEBU), EGF 10 ng/ml (Sigma), insuline 10 µg/ml (Sigma)], de gentamicine 40 µg/ml (Scherring Plough) et de sérum de veau à une concentration de 10 %. Dans ces conditions, on observe une croissance extensive des cellules mononuclées, principalement des myoblastes (confirmé par des études cytochimiques). Après élimination des fragments tissulaires, la couche de myoblastes est dissociée en présence de trypsine (0,25 %) ceci avant la fusion en fibres musculaires ou myotubes. La culture est poursuivie pendant au plus 15 passages et les cellules congelées en azote liquide dans du milieu DMEM contenant 10 % de FCS et 8 % de DMSO.

Pour les transfections, après décongélation, les cellules sont réparties dans des plaques de 96 puits à raison de 5x 103 cellules par puits et cultivées dans des conditions standards pendant 24 heures. avant d'être transfectées en milieu sans sérum par des quantités variables d'ADN plasmidique (2, 1, 0,5, 0,25, 0,125, 0,063, 0,032 ou 0,016 µg) complexé au DC Chol/DOPE seul (à titre de comparaison) ou au mélange DC Chol/DOPE/Chitosan à une concentration en chitosan de 10, 30 ou 60 %. Comme précedemment, on met en oeuvre la préparation pcTG12Nl et les pourcentages de chitosan sont exprimés par rapport à la charge apportée par le DC-Chol. Les différents complexes sont préparés dans du milieu Ham F14 supplémenté en gentamicine et glutamine selon les méthodes conventionnelles ou comme indiqué à l'exemple précédent. Une fois le milieu de culture aspiré, les cellules sont transfectées par 225 µl de chaque échantillon à tester. Quatre heures après la transfection, les cellules sont replacées en présence de sérum par ajout de 25 µl de FCS dans chaque puit. L'activité luciférase est déterminée 48 heures post-transfection après lavage des cellules dans du tampon phosphate et lyse (tampon Proméga). La mesure est réalisée à l'aide d'un luminomètre Microlumat LB96P (Berthold).

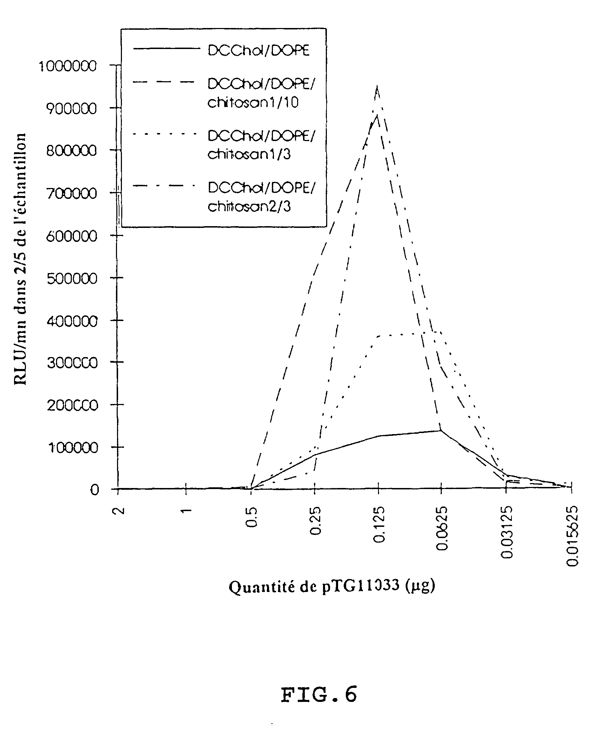

Les résultats reportés dans la Figure 6 montrent que les meilleurs taux de transfection sont obtenus avec les complexes incorporant du chitosan, l'activité luciférase étant 4 à 9 fois plus élevée que lorsque la transfection met en oeuvre l'ADN complexé au DC Chol/DOPE seul. En conclusion, l'effet transfectionnel bénéfique du chitosan a été observé dans une lignée cellulaire d'adénocarcinome de poumon et, à un niveau encore plus élevé, dans une lignée primaire de myoblastes.

### EXEMPLE 6 : Biodistribution des polymères pcTG12 N1, N2 et N3

Les préparations de chitosan pcTG12 N1, N2 et N3 sont marquées à l'iode à l'aide du réactif de Bolton / Hunter (Amersham Life Science ; IM5861) (Bolton et Hunter, 1973, Biochem J. 133, 529-539). Les préparations sont ajustées à 10 mg/ml dans du tampon borate (pour 11: 3,094 g d'acide borique, 3,728 g de chlorure de potassium, pH 6,5) et 500 µl mis en présence de triéthylamine (193 µl pour pcTG12 N1 et 163 µl pour pcTG12 N2 et N3) permettant la déprotonation des groupes -NH₃+ en -NH₂. Chaque polymère ainsi activé est ajouté à 0,5 mCi de réactif de Bolton / Hunter (100 µl) préalablement séché et on laisse la réaction se poursuivre pendant 15 min sur glace.

L'efficacité du marquage est déterminée sur une aliquote de 5 µl par électrophorèse sur papier chromatographique (Whatman, réf. 3001 845) et dans du tampon barbital pH8,6 (Sigma, réf. B6632). On utilise à titre de témoin du Na ¹²⁵I (Amersham, réf. IMS30). La migration des préparations marquées par rapport au témoin permet de quantifier la proportion de ¹²⁵I libre et lié (voir thèse de Ruth Duncan 1979, Keele University). Le pourcentage de ¹²⁵I libre est estimé inférieur à 1 %. Le reste de la préparation est soumis à une dialyse contre une solution de HCl 1 % (v/v) dans de l'eau (tubes de dialyse Sigma D7884) jusqu'à ce que l'on ne puisse plus détecter de radioactivité dans le dialysat.

200 µl de la préparation marquée (5 x 104 cpm) est administrée par voie intraveineuse à des rats Wistar (3 rats par préparation). Les animaux sont sacrifiés 5 min ou 60 min après l'injection et on détermine la radioactivité dans les urines, les faeces et une sélection d'organes après digestion dans la soude NaOH 1N contenant 0,1 % de Triton X-100 (v/v). Le comptage est réalisé à l'aide d'un compteur gamma (LKB Wallac) pendant 10 min par échantillon. La quantité de radioactivité dans chaque organe par rapport à la radioactivité totale est déterminée.

Les résultats présentés dans les tableaux ci-après sont exprimés en pourcentage de la dose récupérée par organe (± la dérivation standard) et montrent une récupération de 32 % (5 min) et 16 % (60 min) des doses injectées pour la préparation NI et 36 % (5 min) et 47 % (60 min) pour la préparation N3.

| Biodistribution de pcTG12 N1 | | |
|---|---|---|
| **Organes** | **5 min** | **60 min** |
| Coeur | 1,3 ± 0,6 | 1,0 ± 0,6 |
| Foie | 13,7 ± 1,3 | 26,5 ± 9,6 |
| Poumons | 27,8 ± 9,0 | 7,2 ± 0,8 |
| Reins | 10,4 ± 1,9 | 11,7 ± 3,3 |
| Rate | 0,8 ± 0,3 | 0,9 ± 0,2 |
| Sang | 45 ± 6,0 | 32,2 ± 10,5 |
| Urine | 0 | 19,1 ± 25,9 |
| Faeces | 0 | 0,2 ± 0,2 |
| Thyroïde | 0,8 ± 0,1 | 1,5 ± 1,0 |

| Biodistribution de pcTG12 N3 | | |
|---|---|---|
| Organes | 5 min | 60 min |
| Coeur | 0,3 ± 0,2 | 0,1 ± 0,1 |
| Foie | 54,7 ± 12,5 | 82,7 ± 1,9 |
| Poumons | 23,5 ± 8,4 | 2,1 ± 1,5 |
| Reins | 5,6 ± 1,0 | 10,5 ± 0,2 |
| Rate | 4,4 ± 1,9 | 2,1 ± 0,1 |
| Sang | 11,4 ± 3,6 | 2,6 ± 0,5 |
| Urine | 0 | 0 |
| Faeces | 0 | 0 |
| Thyroïde | 0,2 ± 0,2 | 0,1 ± 0,1 |

### EXEMPLE 7 : Etude de dégradation des complexes chitosan - ADN

1 mg d'ADN de thymus de veau est complexé à des quantités croissantes des préparations pcTG12 N1, N2 et N3 selon un rapport de charges (polymères / ADN) de 1:1 à 1:0,01. Les complexes ainsi formés sont mis en présence de désoxyribonucléase II (DNAase II) selon le protocole standard et l'état de l'ADN est estimé sur gel d'agarose. La dégradation est notablement réduite (par rapport à un témoin ADN non complexé) lorsque l'ADN est complexé au chitosan selon un rapport de charges élevé.

## Revendications

1. Composition comprenant au moins un composé de formule [X]n dans laquelle n représente un nombre entier de 5 à 300 et X a la formule (1) suivante : 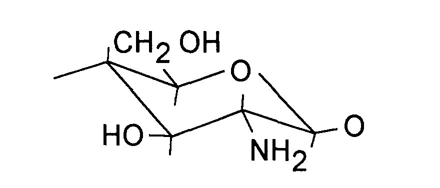 et une substance thérapeutiquement active, **caractérisée en ce que** tout ou partie des résidus X composant ledit composé est modifié par N-, C- et/ou O-alkylation, alcylation, -amino-alkylation et/ou -polyoxyéthylénation.

2. Composition selon la revendication 1, **caractérisée en ce que** n représente un nombre entier de 5 à 150.

3. Composition selon la revendication 2, **caractérisée en ce que** n représente un nombre entier de 5 à 75, et notamment, 5 à 50.

4. Composition selon l'une des revendications 1 à 3, **caractérisée en ce que** ledit composé présente une masse moléculaire moyenne comprise entre 5 et 10 kDa.

5. Composition selon l'une des revendications 1 à 3, **caractérisée en ce que** ledit composé présente une masse moléculaire moyenne supérieure à 10 kDa et inférieure à 50 kDa.

6. Composition selon l'une des revendications 1 à 5, **caractérisée en ce que** ledit composé est sous la forme d'une poly-N-dodécyl-polyglucosamine ou d'une mono-Cα-octadécyl-polyglucosamine.

7. Composition selon l'une des revendications 1 à 6, **caractérisée en ce que** ledit composé est sous forme de sel et plus particulièrement, de chlorure, de sulfate ou de phosphate.

8. Composition selon l'une des revendications 1 à 7, **caractérisée en ce que** ledit composé est substantiellement dépourvu de pigments et d'impuretés résiduelles.

9. Composition selon les revendication 1 à 8, **caractérisée en ce que** ladite substance thérapeutiquement active est un acide nucléique.

10. Composition selon la revendication 9, **caractérisée en ce que** ledit acide nucléique est un vecteur pour l'expression d'un gène d'intérêt thérapeutique placé sous le contrôle des éléments nécessaires à son expression dans une cellule hôte.

11. Composition selon la revendication 10, **caractérisée en ce que** le gène thérapeutique code pour un ribozyme, un ARN antisens ou encore un polypeptide sélectionné parmi une cytokine (interleukine-2, interleukine-12, interferon alpha, béta ou gamma, facteur nécrosant des tumeurs, facteur stimulateur de colonies, antigène du complexe majeur d'histocompatibilité ...), un recepteur de surface cellulaire ou nucléaire, un facteur de coagulation (FVII, FVIII, FIX ...), la protéine CFTR (pour Cystis Fibrosis Transmembrane Conductance Regulator), la dystrophine, l'insuline, une hormone de croissance, une enzyme (rénine, thrombine, uréase ...), un inhibiteur, un antigène viral, un épitope antigénique, un polypeptide supresseur de tumeur, un anticorps, une toxine (thymidine kinase du virus simplex-l de l'herpès, ricine, toxine diphtérique ...), un polypeptide ayant une activité antitumorale ou antivirale et un polypeptide marqueur.

12. Composition selon l'une des revendications 9 à 11, **caractérisée en ce que** le rapport en charge dudit acide nucléique audit composé est compris entre 10/1 et 1/10, et notamment, entre 1/1 et 1/5.

13. Composition selon l'une des revendications 8 à 12, **caractérisée en ce qu'**elle comprend, en outre, au moins un adjuvant capable d'améliorer le pouvoir transfectant de ladite composition.

14. Composition selon la revendication 13, **caractérisée en ce que** ledit adjuvant est un composé amphiphile cationique.

15. Composition selon la revendication 14, **caractérisée en ce que** ledit composé amphiphilique cationique est un composé mono, di ou tri alkylé ou acylé portant de 1 à 4 charges positives.

16. Composition selon la revendication 15, **caractérisée en ce que** ledit composé est un lipide sélectionné parmi le 5-carboxyspermylglycine dioctadécylamide (DOGS), le 3β [N-(N', N'-diméthylaminoéthane)-carbamoyl] cholestérol (DC-Chol), le (2,3-droléylocyl-N-[2(sperminecarboxamido) éthyl] N, N-diméthyl-1-propanaminium trifluoroacétate) (DOSPA), la spermine cholestérol et la spermidine cholestérol.

17. Composition selon l'une des revendications 13 à 16, **caractérisée en ce que** ledit adjuvant est un lipide neutre ou zwitterionique.

18. Composition selon la revendication 17, **caractérisée en ce que** ledit lipide neutre ou zwitterionique est ou dérivé d'une phosphatidyl éthanolamine, phosphatidylcholine, phosphocholine, sphingomyéline, céramide ou cérébroside.

19. Composition selon la revendication 18, **caractérisée en ce que** ledit lipide neutre ou zwitterionique est la dioléyl phosphatidyl éthanolamine (DOPE).

20. Composition selon l'une des revendications 14 à 19, **caractérisée en ce que** le rapport de charge composé amphiphile cationique sur ADN est compris entre 25/1 et 1/1 et de préférence 5/1 et 2/1.

21. Utilisation d'une composition selon l'une des revendications 9 à 20, pour préparer un médicament à but curatif, préventif ou vaccinal destiné au transfert dudit acide nucléique dans une cellule hôte in vivo, ex vivo ou in vitro.

22. Utilisation selon la revendication 21, **caractérisée en ce que** ladite cellule hôte est une cellule de microorganisme, de levure, d'insecte, de plante, humaine, animale et, notamment, mammifère.

23. Utilisation selon la revendication 22,**caractérisée en ce que** ladite cellule hôte humaine est une cellule musculaire ou une cellule épithéliale pulmonaire.

24. Composition comprenant au moins un composé de formule [X]n dans laquelle n représente un nombre entier de 5 à 300 et X a la formule (1) suivante : 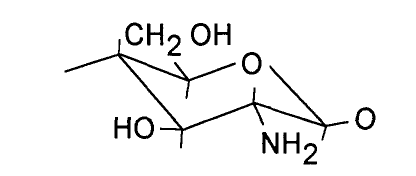 et une substance thérapeutiquement active, **caractérisée en ce que** ledit composé est présent sous forme de sel.

25. Composition selon la revendication 24, **caractérisée en ce que** n représente un nombre entier de 5 à 150.

26. Composition selon la revendication 25, **caractérisée en ce que** n représente un nombre entier de 5 à 75, et notamment, 5 à 50.

27. Utilisation d'une composition selon l'une des revendications 24 à 26 pour préparer un médicament à but curatif, préventif ou vaccinal destiné au transfert de ladite substance thérapeutiquement active dans une cellule hôte *in vivo*, *ex vivo* ou *in vitro*, ladite substance thérapeutiquement active étant un acide nucléique.

28. Composé de formule [X]n dans laquelle n représente un nombre entier de 5 à 300 et X a la formule (1) suivante : 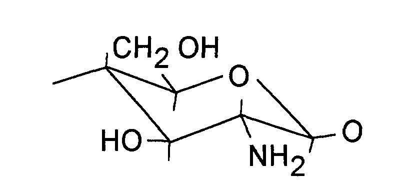 **caractérisé en ce que** tout ou partie des résidus X composant ledit composé est modifié par N-, C- et/ou O-alkylation, alcylation, -amino-alkylation et/ou-polyoxyéthylénation.

29. Composé selon la revendication 28, sous la forme d'une poly-N-dodécyl-polyglucosamine ou d'une mono-Cα-octadécyl-polyglucosamine.

30. Composé de formule [X]n dans laquelle n représente un nombre entier de 5 à 300 et X a la formule (1) suivante : 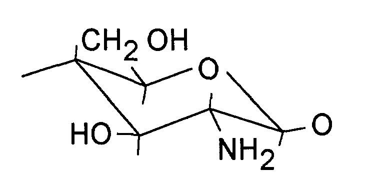 **caractérisé en ce qu'**il est présent sous forme de sel et plus particulièrement, de chlorure, de sulfate ou de phosphate.

31. Composé selon l'une des revendications 28 à 30, **caractérisé en ce que** n représente un nombre entier de 5 à 150.

32. Composé selon la revendication 31, **caractérisé en ce que** n représente un nombre entier de 5 à 75, et notamment, 5 à 50.

33. Composé selon l'une des revendications 28 à 32, **caractérisé en ce qu'**il présente une masse moléculaire moyenne inférieure à 5 kDa.

34. Composé selon l'une des revendications 28 à 32, **caractérisé en ce qu'**il présente une masse moléculaire moyenne comprise entre 5 et 10 kDa.

35. Composé selon l'une des revendications 28 à 32, **caractérisé en ce qu'**il présente une masse moléculaire moyenne supérieure à 10 kDa et inférieure à 50 kDa.

36. Procédé pour préparer un composé de formule [X]n dans laquelle n représente un nombre entier de 5 à 300 et X a la formule (1) suivante : 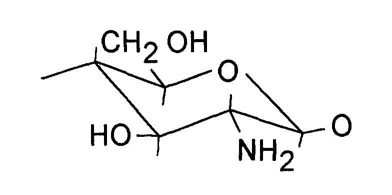 **caractérisé en ce qu'**on soumet une préparation de chitosan de haute masse moléculaire à une hydrolyse acide, une précipitation à l'éthanol et une étape d'extraction des pigments en présence d'un solvant.

37. Procédé selon la revendication 36, **caractérisé en ce que** ledit solvant est du butanol-2.

38. Procédé selon la revendication 36 ou 37, **caractérisé en ce qu'**il comprend après l'étape d'extraction des pigments, une étape de diafiltration différentielle.

39. Procédé selon la revendication 38, **caractérisé en ce que** ladite étape de diafiltration différentielle comprend une première diafiltration à travers une membrane ayant un seuil de coupure de 5 kDa et une seconde diafiltration à travers une membrane ayant un seuil de coupure de 10 kDa réalisée sur le concentré de ladite première diafiltration.

## Patentansprüche

1. Zusammensetzung, enthaltend mindestens eine Verbindung der Formel [X]n, in der n eine ganze Zahl zwischen 5 bis 300 ist, und X der folgenden Formel (1) entspricht: 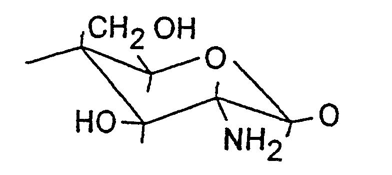 und eine therapeutisch aktive Substanz, **dadurch gekennzeichnet, daß** alle oder Teile der Reste von X, die diese Verbindung bilden, mit N-, C- und/oder O-Alkylierung, Alcylierung, -amino-alkylierung und/oder -polyoxyethylenierung modifiziert sind.

2. Zusammensetzung nach Anspruch 1, **dadurch gekennzeichnet, daß** n eine ganze Zahl von 5 bis 150 ist.

3. Zusammensetzung nach Anspruch 2, **dadurch gekennzeichnet, daß** n eine ganze Zahl von 5 bis 75, insbesondere 5 bis 50 ist.

4. Zusammensetzung nach einem der Ansprüche 1 bis 3, **dadurch gekennzeichnet, daß** die Verbindung eine mittlere Molekularmasse zwischen 5 und 10 kDa aufweist.

5. Zusammensetzung nach einem der Ansprüche 1 bis 3, **dadurch gekennzeichnet, daß** die Verbindung eine mittlere Molekularmasse oberhalb 10 kDa und unterhalb 50 kDa aufweist.

6. Zusammensetzung nach einem der Ansprüche 1 bis 5, **dadurch gekennzeichnet, daß** die Verbindung in Form eines Poly-N-dodecyl-polyglucosamins oder eines Mono-Cα-octadecylpolyglucosamins vorliegt.

7. Zusammensetzung nach einem der Ansprüche 1 bis 6, **dadurch gekennzeichnet, daß** die Verbindung als Salz und insbesondere als Chlorid, Sulfat oder Phosphat vorliegt.

8. Zusammensetzung nach einem der Ansprüche 1 bis 7, charakterisiert dadurch, daß die Verbindung praktisch frei von Pigmenten oder rückständigen Verunreinigungen ist.

9. Zusammensetzung nach einem der Ansprüche 1 bis 8, **dadurch gekennzeichnet, daß** die aktive therapeutische Substanz eine Nucleinsäure ist.

10. Zusammensetzung nach Anspruch 9, **dadurch gekennzeichnet, daß** die Nucleinsäure ein Vector für die Expression eines therapeutisch interessierenden Gens unter der Kontrolle der notwendigen Elemente für seine Expression in einer Gastzelle ist.

11. Zusammensetzung nach Anspruch 10, **dadurch gekennzeichnet, daß** das therapeutische Gen kodiert für ein Ribozym, eine antisense RNA oder ein Polypeptid, ausgewählt unter einem Cytokin (2-Interleukin, 12-Interleukin, Alpha-, Beta- oder Gammainterferon, Nekrosansfaktor von Tumoren, Stimulatorfaktor von Kolonien, Antigen vom komplex höherer Histokompatibilät ...), einen Rezeptor zellulärer oder nucleiner Oberfläche, einen Koagulationsfaktor (FVII, FVIII, FIX ...), das Protein CFTR (für Cystis Fibrosis Transmembrane Conductance Regulator), Dystrophin, Insulin, ein Kreuzungshormon, ein Enzym (Renin, Thrombin, Urease ...), einen Inhibitor, ein virales Antigen, ein antigenisches Epitop, einen Tumorpolypeptidsupressor, einen Antikorpus, ein Toxin (Thymidinkinase des Virus simplex-1 von Herpes, Ricin, Diphterietoxin ...), ein Polypeptid mit antitumoraler oder antiviraler Aktivität und/oder einen Polypeptidmarker.

12. Zusammensetzung nach einem der Ansprüche 9 bis 11, **dadurch gekennzeichnet, daß** das Verhältnis der genannten Nucleinsäure zur genannten Verbindung von 10/1 bis 1/10, insbesondere 1/1 bis 1/5 beträgt.

13. Zusammensetzung nach einem der Ansprüche 8 bis 12, **dadurch gekennzeichnet, daß** sie darüberhinaus mindestens ein Adjuvans enthält, das die transfektierende Wirkung der Zusammensetzung verbessert.

14. Zusammensetzung nach Anspruch 13, **dadurch gekennzeichnet, daß** das Adjuvans eine kationische amphiphile Verbindung ist.

15. Zusammensetzung nach Anspruch 14, **dadurch gekennzeichnet, daß** die kationische amphiphilische Verbindung mono-, di- oder tri-alkyliert oder -acyliert ist und 1 bis 4 positive Ladungen aufweist.

16. Zusammensetzung nach Anspruch 15, **dadurch gekennzeichnet, daß** die Zusammsetzung eines der Lipide 5-Carboxyspermylgycindioctadecylamid (DOGS), 3β [N-(N'Dimethylaminoethan)-carbamoyl] cholesterol (DC-Chol), (2,3-Droleylocyl-N-[2(spermincarboxamido) ethyl] N, N-dimethyl-1-propanaminiumtrifluoracetat) (DOSPA), Spermincholesterol und/oder Spermidincholesterol ist.

17. Zusammensetzung nach einem der Ansprüche 13 bis 16, **dadurch gekennzeichnet, daß** das Adjuvans ein zwitterionisches oder neutrales Lipid ist.

18. Zusammensetzung nach Anspruch 17, **dadurch gekennzeichnet, daß** das zwitterionische oder neutrale Lipid eine der folgenden Verbindungen oder ein Derivat der folgenden Verbindungen ist: Phosphatidylethanolamin, Phosphatidylcholin, Phosphocholin, Sphingomyelin, Ceramid und/oder Cerebrosid.

19. Zusammensetzung nach Anspruch 18, **dadurch gekennzeichnet, daß** das zwitterionische oder neutrale Lipid Dioleylphosphatidylethanolamin (DOPE) ist.

20. Zusammensetzung nach einem der Ansprüche 14 bis 19, **dadurch gekennzeichnet, daß** das Verhälnis von kationischer amphiphiler Verbindung zu DNA von 25/1 bis 1/1, vorzugsweise 5/1 bis 2/1, beträgt.

21. Verwendung einer Zusammensetzung nach einem der Ansprüche 9 bis 20 zur Herstellung eines Medikaments zur Heilung, Prävention oder Impfung, bestimmt zur Übertragung der Nucleinsäure in eine Gastzelle in vivo, ex-vivo oder in vitro.

22. Verwendung nach Anspruch 21, **dadurch gekennzeichnet, daß** die Gastzelle eine Zelle eines Mikroorganismus, eines Hefepilzes, eines Insektes, einer Pflanzes, eines Menschen, eines Tieres und, insbesondere eines Säugetieres ist.

23. Verwendung nach Anspruch 22, **dadurch gekennzeichnet, daß** die menschliche Gastzelle eine Muskelzelle oder eine epitheliale Lungenzelle ist.

24. Zusammensetzung mit Gehalt an mindestens einer Verbindung der Formel [X]n, in der n eine ganze Zahl von 5 bis 300 ist, und X der folgenden Formel (1) entspricht: 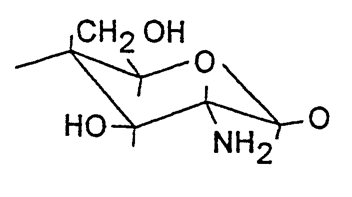 und einer therapeutisch aktiven Substanz, **dadurch gekennzeichnet, daß** die Zusammensetzung in Salzform vorliegt.

25. Zusammensetzung nach Anspruch 24, **dadurch gekennzeichnet, daß** n eine ganze Zahl von 5 bis 150 ist.

26. Zusammensetzung nach Anspruch 25, **dadurch gekennzeichnet, daß** n eine ganze Zahl von 5 bis 75, bevorzugt 5 bis 50 ist.

27. Verwendung einer Verbindung nach einem der Ansprüche 24 bis 26 zur Herstellung eines Medikaments zum Heilen, zur Prävention oder zur Impfung, bestimmt zur Überführung der aktiven therapeutischen Substanz in eine Gastzelle in vivo, ex-vivo oder in vitro, wobei die therapeutische aktive Substanz eine Nucleinsäure ist.

28. Zusammensetzung der Formel [X]n, in der n eine ganze Zahl von 5 bis 300 ist und X der folgenden Formel (1) entspricht: 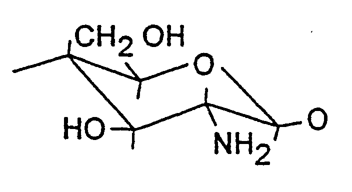 **dadurch gekennzeichnet, daß** alle oder Teile der Reste, die X bilden, mit N-, C- und/oder O-Alkylierung, Acylierung, amino-alkylierung und/oder -polyoxyethylenierung modifiziert sind.

29. Verbindung nach Anspruch 28, in Form eines Poly-N-dodecyl-polyglucosamins oder eines Mono-Cα-ocatadecyl-polyglucosamins.

30. Verbindung der Formel [X]n, in der n eine ganze Zahl von 5 bis 300 ist und X der folgenden Formel (1) entspricht: 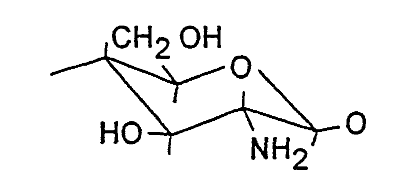 **dadurch gekennzeichnet, daß** sie in Form eines Salzes, insbesondere als Chlorid, Sulfat oder Phosphat vorliegt.

31. Verbindung nach einem der Ansprüche 28 bis 30, **dadurch gekennzeichnet, daß** n eine ganze Zahl von 5 bis 150 ist.

32. Verbindung nach Anspruch 31, **dadurch gekennzeichnet, daß** n eine ganze Zahl von 5 bis 75, bevorzugt 5 bis 50 ist.

33. Verbindung nach einem der Ansprüche 28 bis 32, **dadurch gekennzeichnet, daß** sie eine mittlere Molekularmasse unterhalb 5 kDa aufweist.

34. Verbindung nach einem der Ansprüche 28 bis 32, **dadurch gekennzeichnet, daß** sie eine mittlere Molekularmasse zwischen 5 und 10 kDa aufweist.

35. Verbindung nach einem der Ansprüche 28 bis 32, **dadurch gekennzeichnet, daß** sie eine mittlere Molekularmasse oberhalb 10 kDa und unterhalb 50 kDa aufweist.

36. Verfahren zur Herstellung einer Verbindung der Formel [X]n, in der n eine ganze Zahl von 5 bis 300 ist, und X der folgenden Formel (1) entspricht: 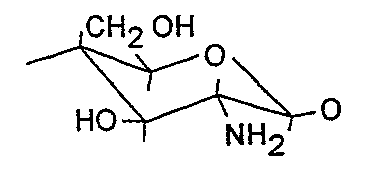 **dadurch gekennzeichnet, daß** man eine Zubereitung von Chitosan hoher Molekülmasse saurer Hydrolyse, Ausfällung in Ethanol und Extraktion von Pigmenten in Gegenwart eines Lösungsmittels unterzieht.

37. Verfahren nach Anspruch 36, **dadurch gekennzeichnet, daß** das Lösungsmittel 2-Butanol ist.

38. Verfahren nach Anspruch 36 oder 37, **dadurch gekennzeichnet, daß** es nach der Extraktion von Pigmenten eine Stufe der differenziellen Diafiltration umfasst.

39. Verfahren nach Anspruch 38, **dadurch gekennzeichnet, daß** die Stufe der differenziellen Diafiltration eine erste Diafiltration über eine Membran einer Trennschwelle von 5 kDa und eine zweite Diafiltration über eine Membran einer Trennschwelle von 10 kDa mit dem Konzentrat der ersten Diafiltration umfasst.

## Claims

1. Composition comprising at least one compound of formula [X]n in which n represents an integer from 5 to 300 and X has the following formula (1): 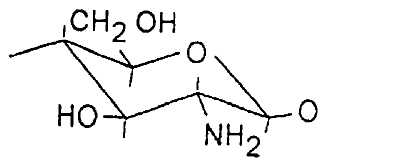 and a therapeutically active substance, **characterized in that** all or part of the X residues constituting said compound is modified by N-, C- and/or O-alkylation, -acylation, -amino-alkylation and/or -polyoxyethylenation.

2. Composition according to Claim 1, **characterized in that** n represents an integer from 5 to 150.

3. Composition according to Claim 2, **characterized in that** n represents an integer from 5 to 75, and in particular 5 to 50.

4. Composition according to one of Claims 1 to 3, **characterized in that** said compound has an average molecular mass of between 5 and 10 kDa.

5. Composition according to one of Claims 1 to 3, **characterized in that** said compound has an average molecular mass greater than 10 kDa and less than 50 kDa.

6. Composition according to one of Claims 1 to 5, **characterized in that** said compound is in the form of a poly-N-dodecyl-polyglucosamine or a mono-Cα-octadecylpolyglucosamine.

7. Composition according to one of Claims 1 to 6, **characterized in that** said compound is in the form of a salt and more particularly a chloride, sulphate or phosphate.

8. Composition according to one of Claims 1 to 7, **characterized in that** said compound is substantially free of pigments and of residual impurities.

9. Composition according to Claims 1 to 8, **characterized in that** said therapeutically active substance is a nucleic acid.

10. Composition according to Claim 9, **characterized in that** said nucleic acid is a vector for the expression of a gene of therapeutic importance placed under the control of elements necessary for its expression in a host cell.

11. Composition according to Claim 10, **characterized in that** the therapeutic gene encodes a ribozyme, an antisense RNA or alternatively a polypeptide selected from a cytokine (interleukin-2, interleukin-12, alpha-, beta- or gamma-interferon, tumour necrosis factor, colony-stimulating factor, antigen of the major histocompatibility complex...), a cell surface or nuclear receptor, a clotting factor (FVII, FVIII, FIX...), CTFR (for Cystic Fibrosis Transmembrane Conductance Regulator) protein, dystrophin, insulin, a growth hormone, an enzyme (renin, thrombin, urease...), an inhibitor, a viral antigen, an antigenic epitope, a tumour suppressor polypeptide, an antibody, a toxin (thymidine kinase of the herpes simplex-1 virus, ricin, diphtheria toxin...), a polypeptide having an antitumour or antiviral activity and a marker polypeptide.

12. Composition according to one of Claims 9 to 11, **characterized in that** the charge ratio of said nucleic acid to said compound is between 10/1 and 1/10, and in particular between 1/1 and 1/5.

13. Composition according to one of Claims 8 to 12, **characterized in that** it comprises, in addition, at least one adjuvant capable of enhancing the transfecting power of said composition.

14. Composition according to Claim 13, **characterized in that** said adjuvant is a cationic amphiphilic compound.

15. Composition according to Claim 14, **characterized in that** said cationic amphiphilic compound is a mono-, di- or tri-alkylated or -acylated compound carrying from 1 to 4 positive charges.

16. Composition according to Claim 15, **characterized in that** said compound is a lipid selected from 5-carboxyspermylglycine dioctadecylamide (DOGS), 3β-[N-(N',N'-dimethylaminoethane)carbamoyl]cholesterol (DC-Chol), (2,3-droleylocyl-N-[2-(sperminecarboxamido)-ethyl]-N,N-dimethyl-1-propanaminium trifluoroacetate) (DOSPA), spermine cholesterol and spermidine cholesterol.

17. Composition according to one of Claims 13 to 16, **characterized in that** said adjuvant is a neutral or zwitterionic lipid.

18. Composition according to Claim 17, **characterized in that** said neutral or zwitterionic lipid is or derives from a phosphatidylethanolamine, phosphatidylcholine, phosphocholine, sphingomyelin, ceramide or cerebroside.

19. Composition according to Claim 18, **characterized in that** said neutral or zwitterionic lipid is dioleylphosphatidylethanolamine (DOPE).

20. Composition according to one of Claims 14 to 19, **characterized in that** the cationic amphiphilic compound to DNA charge ratio is between 25/1 and 1/1 and preferably 5/1 and 2/1.

21. Use of a composition according to one of Claims 9 to 20, for preparing a medicament for curative, preventive or vaccinal purposes intended for transferring said nucleic acid into a host cell in vivo, ex vivo or in vitro.

22. Use according to Claim 21, **characterized in that** said host cell is a microbial, yeast, insect, plant, human, animal add, in particular, mammalian cell.

23. Use according to Claim 22, **characterized in that** said human host cell is a muscle cell or a pulmonary epithelial cell.

24. Composition comprising at least one compound of formula [X]n in which n represents an integer from 5 to 300 and X has the following formula (1): 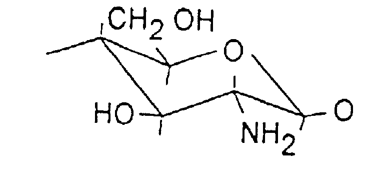 and a therapeutically active substance, **characterized in that** said compound is present in the form of a salt.

25. Composition according to Claim 24, **characterized in that** n represents an integer from 5 to 150.

26. Composition according to Claim 25, **characterized in that** n represents an integer from 5 to 75, and in particular 5 to 50.

27. Use of a composition according to one of Claims 24 to 26 for preparing a medicament for curative, preventive or vaccinal purposes intended for transferring said therapeutically active substance into a host cell *in vivo,* ex *vivo* or in *vitro,* said therapeutically active substance being a nucleic acid.

28. Compound of formula [X]n in which n represents an integer from 5 to 300 and X has the following formula (1): 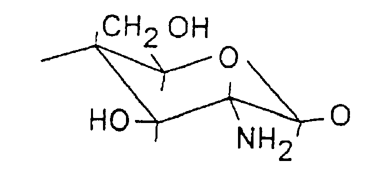 **characterized in that** all or part of the X residues constituting said compound is modified by N-, C- and/or O-alkylation, -acylation, -amino-alkylation and/or -polyoxyethylenation.

29. Compound according to Claim 28, in the form of a poly-N-dodecyl-polyglucosamine or a mono-Cα-octadecyl-polyglucosamine.

30. Compound of formula [X]n in which n represents an integer from 5 to 300 and X has the following formula (1): 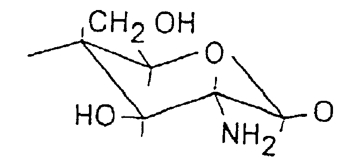 **characterized in that** it is present in the form of a salt and more particularly a chloride, sulphate or phosphate.

31. Compound according to one of Claims 28 to 30, **characterized in that** n represents an integer from 5 to 150.

32. Compound according to Claim 31, **characterized in that** n represents an integer from 5 to 75, and in particular 5 to 50.

33. Compound according to one of Claims 28 to 32, **characterized in that** said compound has an average molecular mass of less than 5 kDa.

34. Compound according to one of Claims 28 to 32, **characterized in that** said compound has an average molecular mass of between 5 and 10 kDa.

35. Compound according to one of Claims 28 to 32, **characterized in that** said compound has an average molecular mass greater than 10 kDa and less than 50 kDa.

36. Method for preparing a compound of formula [X]n in which n represents an integer from 5 to 300 and X has the following formula (1): 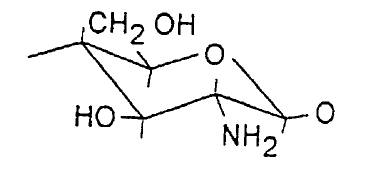 **characterized in that** a preparation of chitosan of high molecular mass is subjected to acid hydrolysis, precipitation with ethanol and a step of extraction of the pigments in the presence of a solvent.

37. Method according to Claim 36, **characterized in that** said solvent is 2-butanol.

38. Method according to Claim 36 or 37, **characterized in that** it comprises, after the step of extraction of the pigments, a step of differential diafiltration.

39. Method according to Claim 38, **characterized in that** said step of differential diafiltration comprises a first diafiltration through a membrane having a cut-off of 5 kDa and a second diafiltration through a membrane having a cut-off of 10 kDa carried out on the concentrate of said first diafiltration.
